# FASCICULE DE BREVET EUROPEEN

(11) **EP 0 321 358 B1**
(45) Date de publication et mention de la délivrance du brevet: **27.07.1994**
(21) Numéro de dépôt: 88403221.0
(22) Date de dépôt: 16.12.1988
(51) Int. Cl.: A61B 5/14

(54) **Dispositif de prélèvement de sang**
Blutentnahmevorrichtung
Blood-sampling device

(30) Priorité: 17.12.1987 FR 8717627
(43) Date de publication de la demande: 21.06.1989
(73) Titulaire: Melet, Francois, F-95520 Osny (FR)
(72) Inventeur: Melet, Francois, F-95520 Osny (FR)
(74) Mandataire: Santarelli, Marc

(56) Documents cités:
- FR-A- 1 242 553
- GB-A- 2 176 710
- US-A- 4 215 702
- US-A- 4 361 155
- US-A- 4 385 637

## Description

La présente invention concerne un dispositif permettant le prélèvement et la conservation du sang en vue d'en effectuer l'analyse gazométrique. La présente invention concerne plus particulièrement un dispositif qui réduit la contamination du sang avec l'air ambiant, qui réduit les forces de contraintes pouvant destructurer l'équilibre gazeux interne du sang et qui réduit la coagulabilité du sang in situ.

De nombreux procédés selon l'art antérieur posent problèmes. Parmi ceux-ci, il existe ceux qui utilisent la forme conventionnelle de seringue avec un corps principal faisant effet de réservoir et un piston pour maitriser la quantité du prélèvement. Ce système, outre qu'il utilise une quantité importante de sang, possède une surface d'échange avec l'air importante entraînant une contamination du sang prélevé. Le désavantage majeur d'un tel système reste cependant la possibilité, de par la présence et la fonction même du piston, de réinjection par l'opérateur de produits dangereux tels que les agents anticoagulants contenus généralement dans la seringue.

D'autres systèmes, selon l'art antérieur, utilisent une forme capillaire. Cette forme permet de recueillir une quantité plus faible de sang. De plus l'échange surfacique air/sang est diminué de par le faible diamètre du capillaire. Ces systèmes ont cependant les désavantages d'utiliser des capillaires en verre qui, de par le matériau même, activent la coagulabilité du sang (phénomène du 'glass-effect'), d'être peu pratiques à transporter et à conserver, et de parfois ne prélever qu'une trop faible quantité de sang pour permettre d'effectuer ultérieurement plusieurs analyses.

Le brevet antérieur US-A-4.361.155 décrit un dispositif de prélèvement du sang avec les caractéristiques indiquées dans la première partie de la revendication 1 et comportant un corps principal tubulaire relié à une aiguille et dans lequel coulissent un piston libre et un plongeur tubulaire actionné de l'extérieur. Un tel dispositif de structure relativement complexe puisqu'elle comprend plusieurs éléments mobiles nécessitant des joints d'étanchéité, présente l'inconvénient de prévoir un arrêt du piston libre dans une position, qui est à la merci d'un déplacement intempestif du plongeur tubulaire accessible de l'extérieur.

L'invention a pour but et performances de surmonter les défauts et problèmes des systèmes décrits précédemment.

Le dispositif de prélèvement du sang artériel en vue d'effectuer une analyse de gazométrie sanguine, selon la présente invention, comprend un corps principal faisant effet de réservoir de sang et comportant une partie antérieure se raccordant à une aiguille de prélèvement du sang et une partie postérieure et dans lequel coulisse un piston libre non accessible à l'opérateur et formé de deux parties concentriques, la partie antérieure des deux parties ayant pour but de réduire au maximum le Volume d'air résiduel à l'état de départ, avant prélèvement du sang artériel et il se caractérise par le fait que le piston libre ne peut se mouvoir que par la pression ou la dépression exercée sur la partie antérieure du corps principal, entre ladite partie antérieure et une partie rétrécie sur laquelle le piston vient en butée en fin de prélèvement, qu'il comporte un filtre poreux à l'air et étanche au sang, jouant le rôle de régulateur de pression et de barrière de contamination du sang et situé dans une partie postérieure du corps principal, et enfin qu'il comporte un bouchon pouvant être placé, en fin de prélèvement, à l'extrémité de la partie postérieure du corps principal pour assurer le transport aisé du sang.

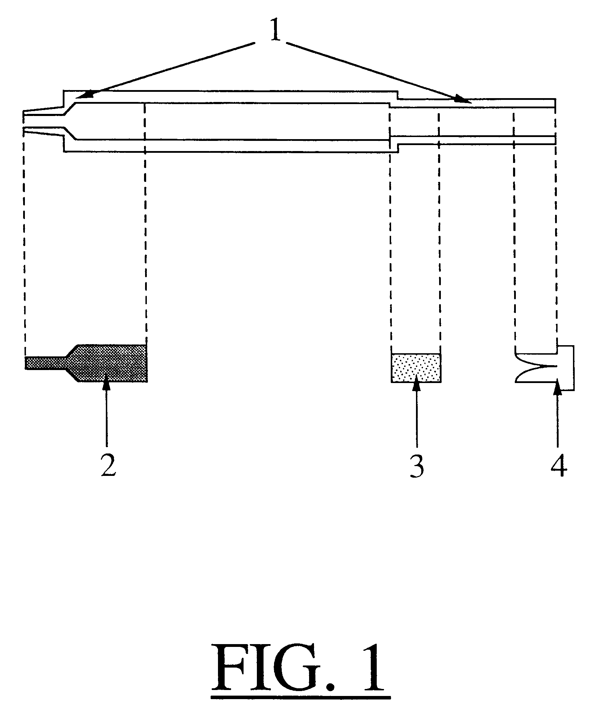

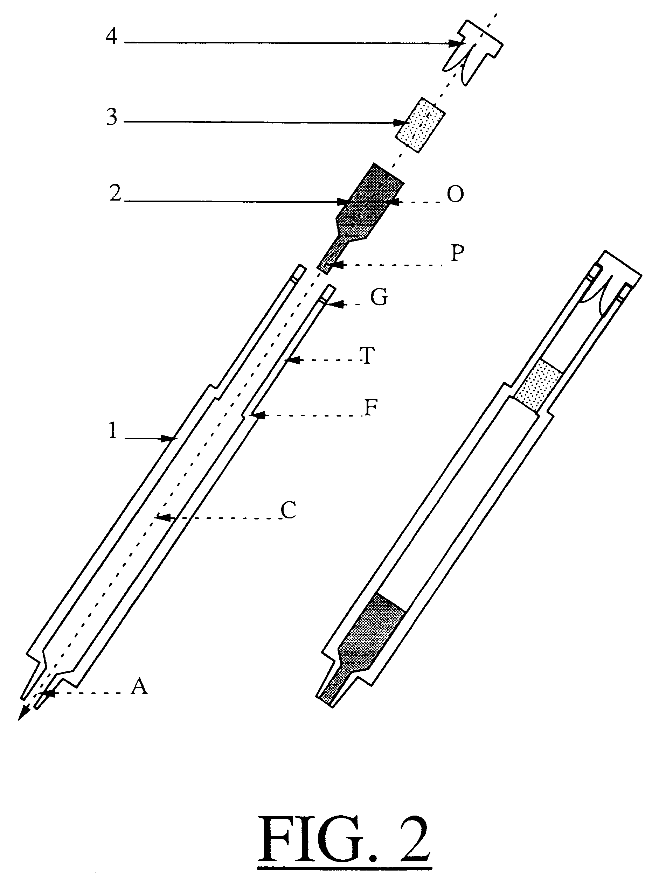

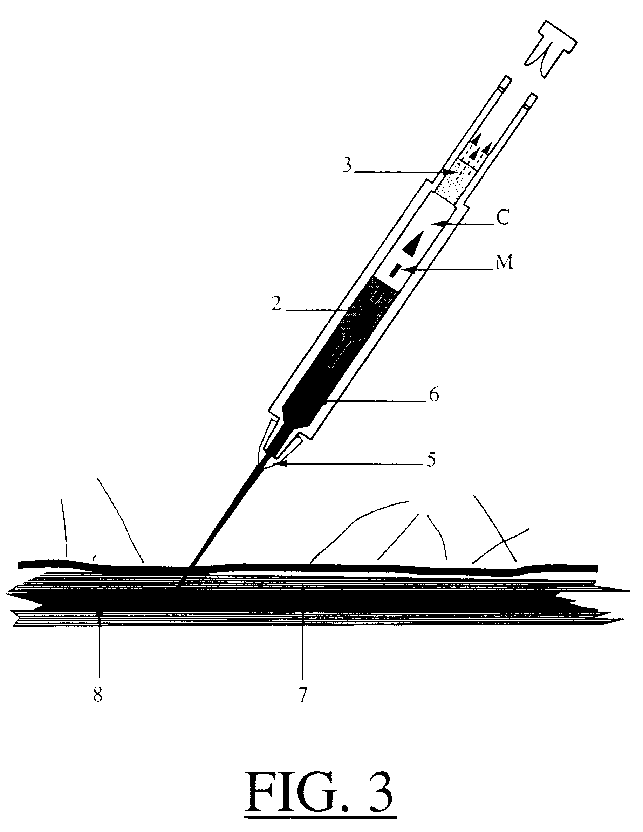

Le système de préférence se compose d'un corps principal (1 Fig.1) faisant effet de contenant du sang, d'un piston cylindrique inerte (2 Fig.1), d'un bouchon cylindrique poreux à l'air et étanche au sang (3 Fig.1), et d'un bouchon plastique (4 Fig. 1). Nous définirons arbitrairement dans la description de l'invention, ci-dessous, comme partie antérieure (A Fig.2) la partie se raccordant à l'aiguille de prélèvement (5 Fig.3) ou partie dans laquelle le sang pénètre dans le système et partie postérieure (T Fig.2) comme la partie opposée et terminale du système.

Le corps principal (1) est formé de cinq parties caractéristiques. Une partie antérieure (A) à laquelle se connecte l'aiguille de prélèvement, une partie centrale (C) formant une chambre dans laquelle se logent le piston et le sang recueilli, une partie rétrécie (F) sur laquelle le piston vient en butée en fin de prélèvement, une partie postérieure (T) dans laquelle le filtre (3) vient se loger et une partie finale au niveau du trou (G Fig.2) sur laquelle un bouchon peut être installé. La partie antérieure (A Fig.2) coaxiale par rapport au système, posséde un diamètre extérieur autorisant le montage d'une aiguille à prélèvement, et un diamètre intérieur d'au moins quinze dixièmes de millimètre. Ce diamètre intérieur minimum est nécessaire pour l'utilisation de l'invention sur des analyseurs gazomètriques de marque INSTRUMENTATION LABORATORY et RADIOMETTER par exemple.

Le piston inerte (2) est formé de deux parties caractèristiques : une partie principale et une partie antérieure (P) coaxiale par rapport à la partie principale (O Fig.2). La partie antérieure (P Fig.2) du piston (2) possède un diamètre inférieur à sa partie principale (o),et très légèrement inférieur aussi au diamètre interne de la partie antérieure (A) du corps principal (1). Avant prélèvement, cette partie cylindrique (P) vient épouser la forme de la partie antérieure (A Fig.2) du corps principal (1), réduisant ainsi le volume mort à l'orifice du système de prélèvement. Ce montage, de type mâle-femelle (Fig.2),a pour avantage de réduire le contact air/sang, réduisant de par là-même la contamination du sang lors de son entrée dans le système. Ce dispositif est fondamental dans l'élaboration de l'invention.

Le filtre poreux (3) placé au niveau de la butée (F Fig.2) dans la partie postérieure (T) du corps principal (1) a pour propriété de laisser passer l'air et de rester étanche au sang. Il permet à l'air comprimé dans la chambre (C Fig.2) du corps principal (1) de s'échapper jusqu'à l'arrivée en butée du piston (2) sur la partie rétrécie (F Fig.2) du corps principal (1) jouant ainsi un rôle de régulateur lors de la montée de l'échantillon sanguin. Ce filtre crée aussi une barrière réduisant la surface d'échanges gazeux entre le sang contenu dans la partie centrale (C) et l'air ambiant situé dans la partie centrale (C) et l'air ambiant situé dans la partie postérieure (T) du corps principal (1) . Ce filtre aura une taille et une porosité adaptées à la pression artérielle du sang prélevé. En effet, il est nécessaire d'avoir une vitesse d'entrée du sang dans le système qui ne soit ni trop grande ni trop faible, permettant ainsi à l'utilisateur de visualiser le remplissage en sang du système. Cette adaptation pourra entraîner des variantes de la longueur de la chambre postérieure (T) du corps principal (1).

Le bouchon (4) est installé à l'extrémité de la partie postérieure (T) du corps principal (1). Un trou (G) situé sur la partie postérieure (T) du corps principal (1) permet de dépressuriser cette partie postérieure lors de la mise en place du bouchon (4). Ce trou (G) sera obstrué en fin de course du bouchon (4). Ce bouchon permet de réduire le volume d'air de la chambre postérieure (T) de la partie principale (1) afin de limiter la contamination dans le temps du sang par l'air. Les tailles en longueur de la partie postérieure (T) du corps principal (1) et celle du filtre (3) sont ajustées pour permettre l'adaptation du bouchon (4) de telle manière que ce dernier, une fois mis en place, vienne se positionner contre le filtre (3). Il permet aussi le transport aisé du système, du site de prélèvement au site d'analyse, sans que le sang puisse ressortir par la partie antérieure (A) du corps principal (1) en position verticale.

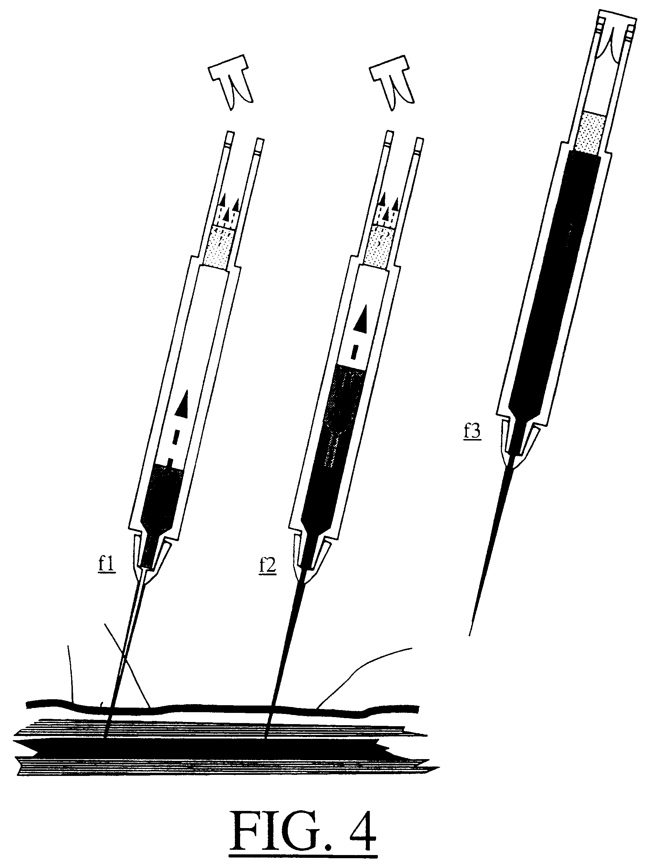

Le protocole d'utilisation du système développe différentes phases : une première phase d'adaptation d'une aiguille stérile (5 Fig.3) sur la partie antérieure (A) du corps principal (1), une deuxième phase (f1 Fig.4) de pénétration de l'aiguille dans le corps du patient (7 Fig.3) jusqu'à l'artère où le sang est à prélever (8 Fig.3), une troisième phase (f2 Fig.4) de montée du sang dans le système de prélèvement, la pression artérielle entraînant une poussée (M Fig.3) du piston (2) jusqu'à la partie formant une butée (F), une quatrième phase (f3 Fig.4) de retrait de l'aiguille, et enfin une dernière phase (f3 Fig.4) de mise en place du bouchon (4) et d'un système de protection conventionnel de l'aiguille.

L'invention posséde l'avantage fondamental de n'offrir aucune possibilité de réinjection vers le malade du contenu de la chambre (C) du corps principal (1). Cet avantage est en effet déterminant dans le coût de fabrication du système puisque ce dernier n'a pas besoin d'être stérile, ce qui évite d'autre part les contraintes administratives relatives à la vente des seringues. L'invention, enfin, fabriquée pour la partie du corps principal (1) et celle du piston (2) en matériau de type polypropylène non mouillable ne déclenche pas la coagulation du sang prélevé. Ce matériau est aussi translucide, ce qui permet une visualisation de la montée du sang dans le système.

Le système concernant l'invention sera utilisé comme un mode de prélèvement capillaire sur les différents analyseurs gazomètriques du marché, c'est-à-dire que le sang sera aspiré par l'analyseur lors d'une mesure de l'échantillon.

## Revendications

1. Dispositif de prélèvement de sang artériel en vue d'effectuer une analyse de gazométrie sanguine comprenant un corps principal (1) faisant effet de réservoir de sang et comportant une partie antérieure (A) se raccordant à une aiguille de prélèvement du sang et une partie postérieure (T) et dans lequel coulisse un piston libre (2) non accessible à l'opérateur et formé de deux parties concentriques (O et P), la partie antérieure (P) des deux parties (O, P) ayant pour but de réduire au maximum le volume d'air résiduel à l'état de départ, avant prélèvement du sang artériel, caractérisé en ce que le piston libre (2), ne peut se mouvoir que par la pression ou la dépression exercée sur la partie antérieure (A) du corps principal (1), entre ladite partie antérieure (A) et une partie rétrécie (F) sur laquelle le piston (2) vient en butée en fin de prélèvement ; en ce qu'il comporte un filtre (3) poreux à l'air et étanche au sang, jouant le rôle de régulateur de pression et de barrière de contamination du sang et situé dans une partie postérieure (T) du corps principal (1), et enfin en ce qu'il comporte un bouchon (4) pouvant être placé en fin de prélèvment à l'extrémité de la partie postérieure (T) du corps principal (1) pour assurer le transport aisé du sang.

2. Dispositif selon la revendication 1, caractérisé en ce que le piston (2) est formé de deux parties coaxiales, l'une des parties de ce piston, la partie principale (O), ayant un diamètre très légèrement inférieur à celui de la partie centrale (C) formant une chambre du corps principal (1), et l'autre des parties du piston, la partie antérieure (P), de diamètre inférieur à celui à la partie principale (O), ayant un diamètre très légèrement inférieur aussi à celui de la partie antérieure (A) du corps principal (1), à laquelle se connecte l'aiguille de prélèvement (5) pour épouser la forme de cette partie antérieure (A) avant prélèvement et réduire ainsi le volume mort à l'orifice du dispositif de prélèvement.

3. Dispositif selon la revendication 1 ou 2, caractérisé en ce que le piston libre (2), situé, avant prélèvement, à la partie antérieure (A) du corps principal (1), peut se déplacer, dans la partie centrale (C) formant chambre du corps principal (1) sous l'action d'une poussée (M) dûe à la pression artérielle agissant sur le piston.

4. Dispostif selon la revendication 3, caractérisé en ce que le déplacement du piston libre (2), à partir de la partie antérieure (A) du corps principal (1) dans la partie centrale (C) formant chambre de ce dernier, s'effectue jusqu'à la partie rétrécie (F) sur laquelle le piston (2) vient en butée, en fin de prélèvement.

## Claims

1. Device for sampling arterial blood for the purpose of carrying out a gasometric blood analysis, comprising a main body (1) acting as a blood reservoir and including a front part (A) connected to a blood sampling needle and a rear part (T) in which slides a free-floating piston (2) which is inaccessible to the operator and which is formed by two concentric parts (O and P), the purpose of the front part (P) of the two parts (O, P) being to reduce to the maximum possible extent the volume of residual air in the initial state, before the sampling of the arterial blood, characterised in that the free-floating piston (2) is able to move only through the positive or negative pressure exerted on the front part (A) of the main body (1), between the said front part (A) and a narrowed part (F) against which the piston (2) comes in abutment at the end of the sampling; in that it includes a filter (3) porous to air and impervious to blood, fulfilling the role of pressure regulator and blood contamination barrier and situated in a rear part (T) of the main body (1), and finally in that it includes a plug (4) which can be put in position at the end of sampling at the end of the rear part (T) of the main body (1) in order to afford easy transportation of the blood.

2. Device according to Claim 1, characterised in that the piston (2) is formed by two coaxial parts, one of the parts of this piston, the main part (O), having a diameter very slightly less than that of the central part (C) forming a chamber in the main body (1), and the other part of the piston, the front part (P), with a diameter less than that of the main part (O), having a diameter also very slightly less than that of the front part (A) of the main body (1), to which the sampling needle (5) is connected, in order to fit into the shape of this front part (A) before sampling and thus reduce the dead space at the orifice of the sampling device.

3. Device according to Claim 1 or 2, characterised in that the free-floating piston (2) situated, before sampling, at the front part (A) of the main body (1), is able to move in the central part (C) forming a chamber in the main body (1) under the effect of a thrust (M) due to the arterial pressure acting on the piston.

4. Device according to Claim 3, characterised in that the movement of the free-floating piston (2), from the front part (A) of the main body (1) in the central part (C) forming a chamber in the latter, takes place as far as the narrowed part (F) against which the piston (2) comes in abutment, at the end of sampling.

## Patentansprüche

1. Vorrichtung zur Entnahme von arteriellem Blut zur Durchführung einer Blutgasometrieanalyse, umfassend einen einen Blutbehälter bildenden Hauptkörper (1), der einen vorderen Teil (A), der an eine Nadel zur Entnahme des Bluts angeschlossen wird, und einen hinteren Teil (T) besitzt und in dem ein für die Bedienungsperson nicht zugänglicher freier Kolben gleitet, der aus zwei konzentrischen Teilen (O und P) gebildet ist, wobei der vordere Teil (P) der beiden Teile (O, P) die Aufgabe hat, das Restluftvolumen im Ausgangszustand vor der Entnahme des arteriellen Bluts maximal zu reduzieren, dadurch gekennzeichnet, daß der freie Kolben (2) sich nur durch den auf den vorderen Teil (A) des Hauptkörpers (1) ausgeübten Druck oder Unterdruck zwischen diesem vorderen Teil (A) und einem verjüngten Teil (F) bewegen kann, an dem der Kolben nach der Entnahme in Anschlag kommt, daß sie ein für Luft poröses und gegenüber Blut dichtes Filter (3) aufweist, das als Druckregler und Sperre gegen Verschmutzung des Bluts dient und in einem hinteren Teil (T) des Hauptkörpers (1) angeordnet ist, und daß sie einen Stopfen (4) aufweist, der nach der Entnahme an dem Ende des hinteren Teils (T) des Hauptkörpers (1) angeordnet werden kann, um eine bequeme Beförderung des Bluts zu ermöglichen.

2. Vorrichtung nach Anspruch 1, dadurch gekennzeichnet, daß der Kolben (2) aus zwei koaxialen Teilen besteht, wobei einer der Teile dieses Kolbens, der Hauptteil (O), einen um sehr wenig kleineren Durchmesser als der eine Kammer bildende mittlere Teil (C) des Hauptkörpers (1) hat und der andere Teil des Kolbens, der vordere Teil (P), dessen Durchmesser kleiner als der des Hauptteils (O) ist, einen ebenfalls um sehr wenig kleineren Durchmesser als der vordere Teil (A) des Hauptkörpers (1), an den die Entnahmenadel (5) angeschlossen wird, hat, um sich an die Form dieses vorderen Teils vor Entnahme anzupassen und so den Totraum an der Öffnung der Entnahmevorrichtung zu reduzieren.

3. Vorrichtung nach Anspruch 1 oder 2, dadurch gekennzeichnet, daß sich der freie Kolben (2), der sich vor der Entnahme im vorderen Teil (A) des Hauptkörpers (1) befindet, in dem eine Kammer bildenden mittleren Teil (C) des Hauptkörpers (1) unter der Wirkung eines Schubs (M) bewegen kann, der durch den auf den Kolben einwirkenden arteriellen Druck erzeugt wird.

4. Vorrichtung nach Anspruch 3, dadurch gekennzeichnet, daß die Bewegung des freien Kolbens (2) in dem eine Kammer bildenden mittleren Teil (C) des Hauptkörpers (1) von dessen vorderen Teil (A) aus bis zu dem verjüngten Teil (F) vor sich geht, an dem der Kolben (2) am Ende der Bewegung in Anschlag kommt.
